# EUROPEAN PATENT SPECIFICATION

(11) **EP 3 436 262 B1**
(45) Date of publication and mention of the grant of the patent: **19.01.2022**
(21) Application number: 17717503.1
(22) Date of filing: 30.03.2017
(51) Int. Cl.: B32B 27/08, B32B 27/30, B32B 27/32, B32B 1/00, B32B 27/18, B32B 3/04

(54) **SINGLE USE REACTOR SYSTEMS AND METHODS**
EINWEG-REAKTOR-SYSTEME UND -VERFAHREN
SYSTÈMES ET PROCÉDÉS DE RÉACTEUR À UN USAGE UNIQUE

(30) Priority: 30.03.2016 US 201662315368 P
(43) Date of publication of application: 06.02.2019
(73) Proprietor: SABIC Global Technologies B.V., 4612 PX Bergen op Zoom (NL)
(72) Inventor: DE BROUWER, Johannes, 5061 AT Oisterwijk (NL); PLANCON, Amy, 6211 GD Maastricht (NL); VAN MIERLOO, Sarah, 6167 RD Geleen (NL)
(74) Representative: Sabic Intellectual Property Group
(86) International application number: PCT/IB2017/051835
(87) International publication number: WO 2017/168372

(56) References cited:
- WO-A1-2012/066126
- JP-A- 2002 194 230
- US-A1- 2011 151 551

## Description

### Technical Field

The disclosure relates to a stabilized polyolefin composition and article formed therefrom, the composition comprising a copolymer of ethylene and at least one α-olefin and α-tocopherol.

### Background

The stabilization of polyolefin composition is known in the art.

Suitable stabilizers known in the art are for example synthetic (poly)phenolic compounds such as tetrakis[methylene-3-(3',5')-di-t-butyl-4-hydroxyphenyl)propionate] methane; octadecyl 3,5-di-t-butyl-4-hydroxyhydrocinnamate; 1,1,3-tris(2-methyl-4-hydroxy-5-t-butylphenyl)butane; 1,3,5-trimethyl-2,4,6-tris(3,5-di-t-butyl-4-hydroxybenzyl)benzene, bis-[3,3-bis-(4'-hydroxy-3'-t-butylphenyl butanoic acid]-glycol ester; tris(3,5-di-t-butyl-4-hydroxy benzyl)isocyanurate; 1,3,5-tris(4-t-butyl-2,6-dimethyl-3-hydroxybenzyl)isocyanurate; 5-di-t-butyl-4-hydroxy hydrocinnamic acid triester with 1,3,5-tris(2-hydroxyethyl)-s-triazine-2,4,6(1H, 3H, 5H)trione; p-cresol/dicyclopentadiene butylated reaction product and 2,6-bis(2'-bis-hydroxy-3'-t-butyl-5'-methyl-phenyl-4-methyl-phenol).

The stabilized polyolefin compounds may be processed via for example injection molding, blow molding, extrusion molding, compression molding or thin-walled injection molding techniques. The obtained products may be applied in a huge amount of applications for example in food contact packaging applications, biomedical applications, health care applications or pharmaceutical applications.

Most of the synthetic (poly)phenolic antioxidants used are strictly regulated by governments because they are suffering from serious limitations. Synthetic antioxidants can thereby diffuse into the surrounding medium. This can in turn lead to contamination of food and/or other human-used products with potentially toxic by-product substances. This problem may arise simply because some antioxidants are toxic above a certain level of concentration.

Accordingly, the use of such antioxidants is already an issue in for example food- and water contact applications, in medical and pharmaceutical devices. With increased consumer concerns about the amount of chemicals in their foods, processors are looking for improved ways to protect their products. US20110151551 discloses bio-reactors.

### Summary

With improvements in biotechnology and the shift from small-molecule to large-molecule biologic drugs that are used by smaller volumes of customers, there is a need for flexible and scalable facilities for biopharmaceutical production processes that can produce more than one product offering (as opposed to traditional large-scale stainless-steel systems or glass systems).

Single-Use Systems allow smaller production runs with improvements in yield, faster cleaning and sterilization, shorter implementation time, and increased speed to market in biologic drug production.

In certain embodiments, the present disclosure relates to a single use system. Some characteristics of single use systems are (1) flexibility and (2) avoidance of cleaning, sterilization, and validation. This results in advantages such as cost reduction, reduced utilities requirements, improved sustainability, shorter development cycles and higher outputs per unit volume.

Standard fixed in place process installations are designed for a particular type of reaction or synthesis and are best not used for other purposes. If process units need to be added, removed or modified, this will involve tinkering which introduces high cost through labor and productivity loss. Such installations are best used for (continuous) production of one single product. Discontinuous or batch production as is often the case in biopharmaceutical process requires cleaning and sterilization in between production batches followed by validation of the cleaning step efficacy. Such processes also add cost through labor, productivity loss and usage of water and cleaning chemicals, which, in addition, have a negative environmental impact.

Single use systems (SUS) made from plastic are available on the market. Conventional systems often consist of various components that can be combined into a configuration that is suitable for the target reaction. The systems are complemented by placeholders that keep the single use components in the required shape and a variety of other reusable modular equipment to support process operations such as e.g. shakers. The plastic components are made from e.g., polyethylene or polyvinylidene fluoride films and additional non-contact layers may be added for enhancing barrier properties (such as ethylene vinyl alcohol) or providing more strength (elastomeric thermoplastic, TPU).

Commercial plastics are complex mixtures of different components. There is variation in chain length and composition between the individual polymer chains in the plastic material. Components may have been added for modification of the properties (e.g. fillers, rubbers) or to facilitate processing (e.g. stabilizers, release agents). Finally, impurities from the production process can be present. These may have come in with the raw materials that have been used, or they may have been generated by side reactions, heating, irradiation, hydrolyses, sterilization and other processes. In the application as single use system, such components may leach into the reaction mixture and influence the reactions taking place.

For one particular impurity [bis(2,4-di-tert-butylphenyl)phosphate], the breakdown product of the commonly used plastics heat stabilizer tris(2,4-di-tert-butylphenyl)phosphite (commercially available under the tradename Irgaphos^{®} 168 from BASF), it has been found that it has a strong effect on cell growth rate in Chinese hamster ovary (CHO) cell cultures, either reducing that rate or bringing production to a stop, depending on the concentration. ("A Cytotoxic Leachable Compound from Single-Use Bioprocess Equipment that Causes Poor Cell Growth Performance", Hammond et al., Biotechnology Progress, Volume 30, Issue 2, 2014, Pages 332-337).

The storage and reaction bags are key SUS components. Linear low density polyethylene (LLDPE) is a frequently used material for these components due to its low price, suitable mechanical properties and broad solvent compatibility. LLDPE is a substantially linear polymer (polyethylene), with significant numbers of short branches, commonly made by copolymerization of ethylene with longer-chain olefins. In general, LLDPE is produced at lower temperatures and pressures by copolymerization of ethylene and such higher alpha olefins as butene, hexene, or octene. The copolymerization process produces an LLDPE polymer that has a narrower molecular weight distribution than conventional low density polyethylene, and in combination with the linear structure, significantly different rheological properties. Many LLDPE grades, however, have not been designed for the specific application of SUS and may contain tris(2,4-di-tert-butylphenyl)phosphite, its breakdown product bis(2,4-di-tert-butylphenyl)phosphate and/or have a level of extractables that has not been optimized or controlled.

This requires end-users to spend more effort in qualifying SUS components.

In contrast to metal or glass reactor systems, plastic reactor system may leech components into the media that are in contact with them during reaction, storage or transport. Such leeched components may contaminate the reactor contents or influence the processed that are conducted in the reactor system. It has been found that bis(2,4-di-tert-butylphenyl)phosphate, a breakdown product of the frequently used polymer stabilizer tris(2,4-di-tert-butylphenyl)phosphite reduces cell growth rates in CHO cell growth cultures. Though the effect may be different with other cell growth cultures it is undesirable for biopharmaceutical companies to evaluate such potential effects before use. Therefore, there is a clear need for single use reactor systems which are free from bis(2,4-di-tert-butylphenyl)phosphate and from stabilizer tris(2,4-di-tert-butylphenyl)phosphite.

There is a continuous need to provide improved stabilized polyolefin compositions having no dangerous effects when dispersed in the environment and which also fulfill all requirements related to processing or short term heat stabilization.

The disclosure, in certain embodiments, relates to a single use reaction system. Such systems are increasingly used in the (bio)pharmaceutical industry for synthesis, cell growth and (bio)reactions. There are many advantages attached to the use of such systems, but one risk that comes with the use of plastics is that components from the plastic may leach into the reaction medium. This disclosure describes a single use system made from a plastic which is free of bis(2,4-di-tert-butylphenyl)phosphate and its precursor tris(2,4-di-tert-butylphenyl)phosphite and hence does not require users to test for negative effects on productivity of such components. As referenced herein the materials and methods referencing a bioreactor or bioreactor bag may be applied to other articles such as two- and three-dimensional storage bags, mixing bags, shipping bags, freeze/thaw bags, final filling bags, sampling bags, and components such as tubing, filters, liners, valves.

One or more of the system components may be formed from the disclosed compositions. In certain embodiments, each component of a system may include the same composition.

The disclosure is characterized in that the stabilized polyolefin composition comprises and/or consists of:
- A. a copolymer of ethylene and at least one α-olefin, and
- B. between 25 and 300 ppm by weight of α-tocopherol relative to the component A.

The use of this composition results in the ability to introduce a bio-inspired antioxidant package with excellent resistance to oxidative degradation. As used herein, bio-inspired connotes synthetic materials whose structure, properties, or function mimic those of natural materials or living matter. Some examples of bio-inspired materials include light-harvesting photonic materials that mimic photosynthesis, structural composites that imitate the structure of nacre, and metal actuators inspired by the movements of jellyfish. The use of articles including the compositions described herein may minimize the reliance on bis(2,4-di-tert-butylphenyl)phosphate and its precursor tris(2,4-di-tert-butylphenyl)phosphate. Additional advantages of the stabilizer composition according to the disclosure are the presence of less discoloration of the material after recycling. Furthermore the stabilizer composition according to the disclosure results in improved processing stability and/or improved organoleptic properties.

### Detailed Description

The stabilized polyolefin composition is efficiently stabilized, while limiting and/or avoiding the use of toxic substances. This may especially for example allow the improvement of processing stability, especially by reducing cross-linking during extrusion and/or multiple extrusions

A further advantage is that the stabilizer composition according to the disclosure results in improved organoleptic properties. Moreover, the risk related to and/or the amount of compound(s) possibly migrating out of the stabilized polyolefin composition may be reduced.

Component A may be a copolymer of ethylene with at least one α-olefin, preferably a LLDPE. LLDPE may, for example, be obtained by polymerizing ethylene with at least one α-olefin, which may be selected from 1-butene, 1-pentene, 4-methyl-1-pentene, 1-hexene, 1-heptene and/or 1-octene, preferably 1-butene.

For example, the polymerized α-olefin may for example account for between 0.05 w% and 15 w%, preferably between 0.1 w% and 12 w%, further preferred between 1 w% and 10 w% , further preferred between 0.1 w% and 5 w% or between > 5 w% and < 10 w% compared to the total weight of the LLDPE.

A copolymer of ethylene and at least one α-olefin may for example have a density as determined according to ISO 1183-1 (2012), method A of ≥ 910 kg/m³ and ≤ 940 kg/m³, preferably between 913 kg/m³ and 923 kg/m³. This may lead to mechanical properties suitable for the use for film applications.

A copolymer of ethylene and at least one α-olefin may for example have an MFI represented by the corresponding melt mass flow rate (MFR) as measured according to ISO 1131-1 (2011) at 190°C and at a load of 2.16 kg of between 0.1 dg/min and 10 dg/min, preferably between 0.5 dg/min and 5 dg/min, further preferred between 0.5 dg/min and 3 dg/min, even further preferred >0.5 dg/min and < 2 dg/min. This may lead to a processability suitable for the use for film applications.

LLDPE may preferably be produced using a gas phase or slurry process. The production processes of polyethylenes are summarized in "Handbook of Polyethylene" by Andrew Peacock (2000; Dekker; ISBN 0824795466) at pages 43-66.

Component B may be α-tocopherol.

According to the disclosure the α-tocopherol may be preferably synthetic α-tocopherol.

A suitable example of α-tocopherol and/or synthetic α-tocopherol according to the disclosure may be is Irganox^{®} E 201 (supplied by BASF) which is a racemic mixture of equal amounts of all eight possible stereoisomers of α-tocopherol (RRR, SSS, RRS, RSR, SSR, SRS, SRR, RSS) and is referred to as dl-α-tocopherol or all-rac-alpha-tocopherol.

The use of α-tocopherol may for example allow efficient stabilization with relatively low loadings of α-tocopherol.

The stabilized polyolefin composition may preferably not comprise other tocopherols and/or no tocotrienol and/or no natural vitamin E and/or no other antioxidant and/or no other compound comprising at least one phenolic motif. This may allow an efficient stabilization of the stabilized polyolefin composition, while reducing the risk associated with and/or the amount of compounds that may migrate out of the stabilized polyolefin composition. This may thus reduce possible health hazards.

The amount of component B ranges between 25 ppm and 300 ppm by weight, preferably between 50 ppm and 200 ppm by weight, further preferred between > 50 ppm and <100 ppm by weight, relative to the component A. This may allow an efficient stabilization of the stabilized polyolefin composition, while reducing the risk associated with and/or the amount of compounds that may migrate out of the stabilized polyolefin composition. This may thus reduce possible health hazards.

Besides components A and B, the stabilized polyolefin composition according to the disclosure may also comprise component C, which may be at least one organometallic stearate such as for example magnesium stearate, aluminum stearate, sodium stearate and calcium stearate and/or at least one inorganic hydrotalcites, such as for example DHT4A , preferably calcium stearate. An organometallic stearate and/or an inorganic hydrotalcite may improve processability.

The amount of optional component C may range between 100 ppm and 1000 ppm by weight, more preferably between 200 ppm and 800 ppm by weight, further preferred between 400 ppm and 600 ppm by weight, relative to the component A.

The weight ratio B:C may range for example between 0.05:1 and 0.5:1, preferably 0.07:1 to 0.3:1, further preferred between 0.1:1 and 0.2:1.

Another advantage of the stabilized polyolefin composition may be an improved color retention after recycling.

A stabilized polyolefin composition according to the disclosure may preferably for example comprise and/or consist of:
- A. a copolymer of ethylene and at least one α-olefin,
- B. between 25 and 300 ppm by weight of α-tocopherol relative to the component A and
- C. between 100 ppm and 1000 ppm by weight of a stearate, preferably calcium stearate, relative to the component A.

That the stabilized polyolefin composition consists of the components listed above may thereby preferably mean that no other antioxidant and/or no other stearate or hydrotalcite and/or no other compound/component is present.

The composition according to the disclosure may be used in the production of specific articles. Examples of preferred articles are films and/or pouches, especially for packaging applications such as food and/or beverage packaging applications, for biomedical applications for example in-vivo applications, health care applications and/or pharmaceutical applications, medical applications, especially films and/or pouches for biomedical and/or health care, and/or pharmaceutical and/or medical applications.

The present disclosure also concerns the use of a stabilized polyolefin composition according to the disclosure for films and/or pouches, especially for packaging applications such as food and/or beverage packaging applications, for biomedical applications for example in-vivo applications, health care applications and/or pharmaceutical applications, medical applications, especially films and/or pouches for biomedical and/or health care, and/or pharmaceutical and/or medical applications.

The present disclosure also concerns use of a stabilized polyolefin composition a bioreactor such as a bag, pouch, or chamber configuration. As an example, the bioreactor may include at least one sheet of polymer material formed from a composition comprising: A. a copolymer of ethylene and at least one α-olefin; and B. between 25 and 300 ppm by weight of α-tocopherol relative to the component A.

In certain embodiments, a bioreactor bag may be an inflatable bag and may comprise a top sheet of polymer material; and a bottom sheet of the polymer material coupled along at least one edge of the top sheet to form a sealed bag, wherein the polymer material is formed from a composition comprising: A. a copolymer of ethylene and at least one α-olefin; and B. between 25 and 300 ppm by weight of α-tocopherol relative to the component A. The top sheet and the bottom sheet may be formed from separate sheets or may be formed from a contiguous sheet folded at least partially upon itself. Other configurations of material may be used. The bioreactor may comprise multiple layer sheets or films including 2, 3, 4, 5 or more layers of material or gaseous spacer layers. The bioreactor bag may further comprise: C. a stearate. The copolymer of ethylene and at least one α-olefin is LLDPE.

The amount of component B may range between > 50 ppm and <150 ppm by weight relative to the component A. The amount of component C may range between 100 ppm and 1000 ppm by weight relative to the component A.

In certain embodiments, the stabilized polyolefin composition may be used to make a film. This may be a homogeneous film from the stabilized polyolefin composition, but may include a multilayer film in which the various layers have a different function and in which at least one of the outer layers is made from the stabilized polyolefin composition. In certain embodiments, products derived from the film such as bags are then designed and produced in such a way that the layer consisting of the bio-stabilized polyolefin composition is on the inside of the bag such that this is the layer that is in contact with the reaction medium or the storage solution or the (bio)pharmaceutical product. In other embodiments, products derived from the film such as bags are then designed and produced in such a way that the layer consisting of the bio-stabilized polyolefin composition containing a natural based stabilizer, this polyolefin is on the outside of the bag.

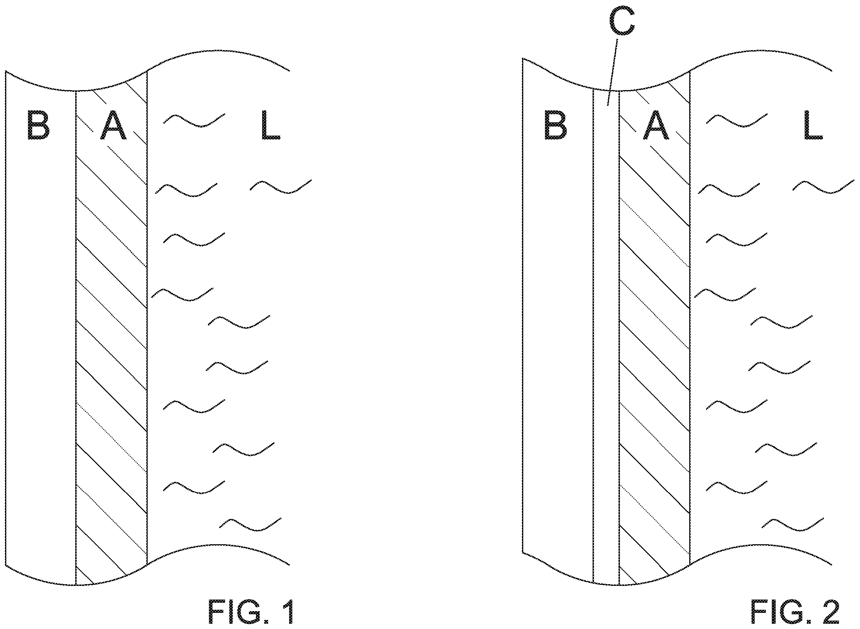

As an illustrative example, when a multilayer film is used the structure may be as depicted in Figure 1 where the film has two functional layers. Layer A is the 'contact layer' which is made from the stabilized polyolefin composition. This is the layer that is in contact with the reaction medium L. Layer B is the 'outer layer' which may be from the same or from a different composition as A and have the same or a different thickness as A. The composition and thickness of layer B can be chosen such that it enhances the certain properties of the film such as UV resistance, strength, rigidity, puncture resistance, gas permeability, radiation resistance and/or heat resistance.

In between the contact layer A and the outer layer B, additional layers may be added. Figure 2 shows a three layered film having one intermediate functional layer C. One or more of such intermediate layers may be used to further enhance the performance of the film. Layer C can for instance be an ethylene vinylalcohol copolymer which reduces gas permeability.

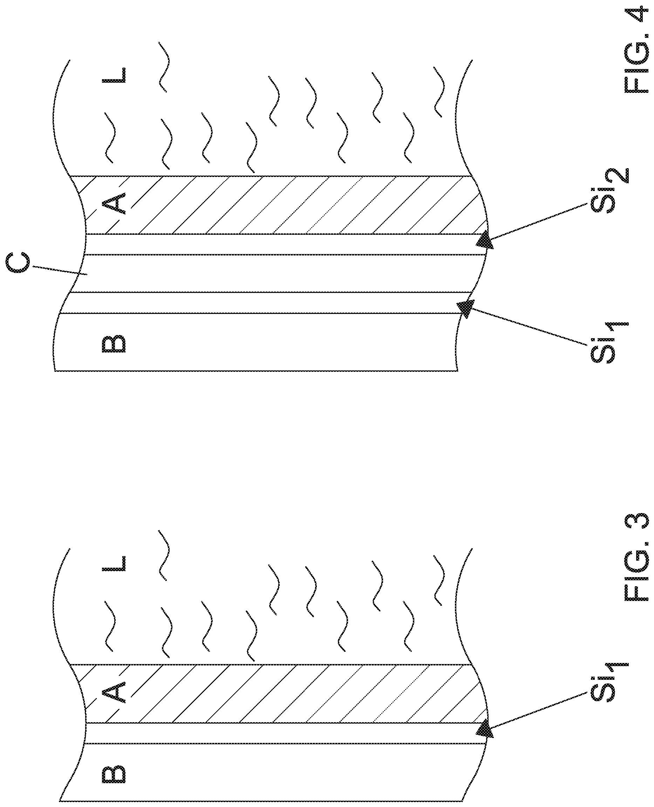

In addition to functional layers (A, B, C), also structural layers may be added. Structural layers improve the adhesion between the functional layers and prevent film failure through delamination. Such structural layers may be made from an adhesive. Figures 3 and 4 show multifilm layers with 2 and three functional layers having structural layers (S1, S2) in between.

The films can be used to manufacture SUS components. The films are particularly well suited to make bags. Such bags may be used for storage and/or for executing (bio)chemical reactions.

The bags can be made in a variety of sizes to suit different process steps and scale-up stages. Bag volumes may range from 0.1 L to 5.0 L for laboratory settings up to 10,000 L for pilot, pre-production and production scales.

### Examples

The disclosure will now be elucidated by way of the following examples without however being limited thereto. Without further elaboration, it is believed that one skilled in the art can, using the description herein, utilize the present disclosure. The following examples are included to provide addition guidance to those skilled in the art of practicing the claimed disclosure. The examples provided are merely representative of the work and contribute to the teaching of the present disclosure. Accordingly, these examples are not intended to limit the disclosure in any manner.

While aspects of the present disclosure can be described and claimed in a particular statutory class, such as the system statutory class, this is for convenience only and one of skill in the art will understand that each aspect of the present disclosure can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

The following examples are put forth so as to provide those of ordinary skill in the art with a complete disclosure and description of how the methods, devices, and systems disclosed and claimed herein are made and evaluated, and are intended to be purely exemplary and are not intended to limit the disclosure. Efforts have been made to ensure accuracy with respect to numbers (e.g., amounts, temperature), but some errors and deviations should be accounted for. Unless indicated otherwise, parts are parts by weight, temperature is in degrees Celsius (°C) or is at ambient temperature, and pressure is at or near atmospheric.

The following compounds were used:
- LLDPE: SABIC^{®} LLDPE 118NE (without additives (no stabilizers, no acid scavengers)
- α-tocopherol purchased from BASF (Irganox^{®} E 201)
- calcium stearate PLC (vegetable based) supplied by Faci S.p.A.

### Multiple Extrusion Experiments:

A Leistritz Micro 27/GL24 Schnecke 10 was used under air. Multiple-pass extrusion involves repeatedly passing the polymer through an extruder and then collecting the samples after each pass. After the compounding extrusion (first extrusion step), the pellets were re-extruded five more times with samples being taken after each pass through the extruder.

### Melt Flow Index Measurements:

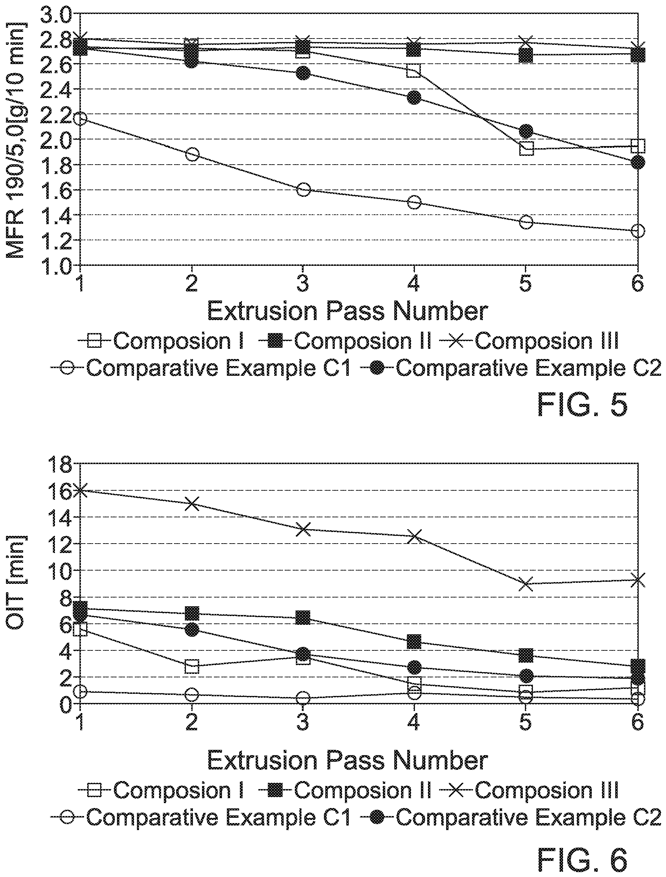

Melt stability was characterized after each extrusion step using melt flow index measurements (MFI) by measuring the MFR after each extruder pass. MFI was measured by applying a standard load of 2.16 kg at a melt temperature of 190°C, in accordance with ASTM D 1238-13. Results are shown below in Fig. 5.

### Oxidation Induction Time Measurements:

Oxidation Induction Time (OIT) has been measured after each extrusion step according to ISO 11357-6: Plastics -- Differential scanning calorimetry (DSC) -- Part 6:
Determination of oxidation induction time (isothermal OIT) in duplicate.
Temperature: 200°C
Heating rate: 20°C/min
Results are shown below in Fig. 6.

### Examples I to V and comparative example C1 and C2

Table 1 shows a comparison between stabilized polyolefin compositions according to the disclosure (I, II, III, IV, and V) and comparative examples C1 and C2.

**Table 1**

| | | Concentration versus LLDPE |
|---|---|---|
| C1 | No additives | 0 |
| I | α-tocopherol | 50 ppm |
| II | α-tocopherol | 150 ppm |
| III | α-tocopherol | 200 ppm |
| C2 | Irganox^{®} 1076 + Irgafos^{®} 168 | 200 ppm + 800 ppm |
| IV | α-tocopherol | 75 ppm |
| V | α-tocopherol | 150 ppm |

Irganox^{®} 1076 is octadecyl 3,5-di-t-butyl-4-hydroxyhydrocinnamate and Irgafos^{®} 168 is tris (2,4-di-t-butylphenyl) phosphite.

Figures 5-6 illustrate comparative graphs. Comparison of compositions I to III with comparative examples C1 and C2 shows that processing stability for the compositions according to the disclosure is better than for C 1 and/or comparable or better than for C2, in that the MFR does not significantly decrease after two consecutive extrusions, preferably even after 3 consecutive extrusions and for composition II and III even after 6 consecutive extrusions. This means that cross-linking occurring during extrusion may be reduced.

Moreover, OIT can be increased for the compositions I to III compared to C1 and/or for compositions II and III even compared to C2. Furthermore, an increased OIT may also be maintained over several consecutive extrusions.

Each of the component materials disclosed herein are either commercially available and/or the methods for the production thereof are known to those of ordinary skill in the art. It is understood that the compositions disclosed herein have certain functions. Disclosed herein are certain structural requirements for performing the disclosed functions and it is understood that there are a variety of structures that can perform the same function that are related to the disclosed structures, and that these structures will typically achieve the same result.

The following observations were made with respect to the optical quality of materials described in this disclosure. To measure of the optical film quality of the material, a gel count evaluation was performed. Gel count is used in order to determine the gel level in polyethylene film. Gels are optical irregularities in the film which are more predominant if heat stabilization is poor. When measuring gel count, a cast film of 25 µm is produced from the material. This film is inspected via an optical system in order to determine defects within the film. These defects are measured, classified in size (>300, >450, >600µm) and quantified in number of gel class per m² of film. The equipment used for the data included herein was a Göttfert extruder V-type (O30 mm, 20L/D).

The extrusion settings applied during the gel count analysis provided in Tables 3 and 4 are included in Tables 2A and 2B, respectively, below.

**Table 2A**

| | Gelcount method |
|---|---|
| Temperature window (°C) | 170-2^{∗}180-2^{∗}170-2^{∗}175-185 |
| Film thickness (µm) | 25 |
| Rpm (rpm) | 40 |
| Amount material (m²) | ~20 |
| Nitrogen blanket | No |
| Throughput | ~3.6 kg/h |
| Extruder type | Göttfert Extruder P-Serie D=30 mm; L/D 20 |

**Table 2B**

| | Gel count method |
|---|---|
| Temperature window (°C) | 160-180-210-5^{∗}230 |
| Film thickness (µm) | 25 |
| Rpm (rpm) | 40 |
| Amount material (m2) | ~40 |
| Nitrogen blanket | No |
| Throughput | ~3.6 kg/h |

Table 3 shows the result of gel count measurements of compositions C2 compared with compositions IV and V.

**Table 3**

| Gel size | C2 | IV | V |
|---|---|---|---|
| >300 µm | 7.7 | 714.0 | 23.5 |
| >450 µm | 1.4 | 165.7 | 6.5 |
| >600 µm | 0.2 | 50.5 | 2.4 |

Table 4 shows the result of gel count measurements of compositions V compared with composition C2. Gels have been counted on 25µm film cast at 230°C and are expressed as gels/m² after having been counted on 40m². If processing takes place at higher temperatures such as those used in the production of these films, a higher level of stabilizer such as composition V is desired.

**Table 4**

| Gel Size | C2 | V |
|---|---|---|
| >300 µm | 9.7 | 2.6 |
| >450 µm | 1.6 | 1.1 |
| >600 µm | 0.3 | 0.6 |

### Definitions

It is to be understood that the terminology used herein is for the purpose of describing particular aspects only and is not intended to be limiting. As used in the specification and in the claims, the term "comprising" may include the aspects "consisting of' and "consisting essentially of." Unless defined otherwise, all technical and scientific terms used herein have the same meaning as commonly understood by one of ordinary skill in the art to which this disclosure belongs. In this specification and in the claims which follow, reference will be made to a number of terms which shall be defined herein.

As used in the specification and the appended claims, the singular forms "a," "an" and "the" include plural referents unless the context clearly dictates otherwise. Thus, for example, reference to "a polycarbonate" includes mixtures of two or more such polycarbonates. Furthermore, for example, reference to a filler includes mixtures of two or more such fillers.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "optional" or "optionally" mean that the subsequently described event, condition, component, or circumstance may or may not occur, and that the description includes instances where said event or circumstance occurs and instances where it does not.

As used herein, the term or phrase "effective," "effective amount," or "conditions effective to" refers to such amount or condition that is capable of performing the function or property for which an effective amount is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, it should be understood that an appropriate effective amount will be readily determined by one of ordinary skill in the art using only routine experimentation.

Disclosed are component materials to be used to prepare disclosed compositions of the disclosure as well as the compositions themselves to be used within methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the disclosure. Thus, if there are a variety of additional steps that can be performed it is understood that each of these additional steps can be performed with any specific aspect or combination of aspects of the methods of the disclosure.

References in the specification and concluding claims to parts by weight, of a particular element or component in a composition or article denotes the weight relationship between the element or component and any other elements or components in the composition or article for which a part by weight is expressed. Thus, in a composition containing 2 parts by weight of component X and 5 parts by weight component Y, X and Y are present at a weight ratio of 2:5, and are present in such ratio regardless of whether additional components are contained in the compound.

## Claims

1. An inflatable bioreactor bag comprising:
a top sheet of polymer material; and
a bottom sheet of the polymer material coupled along at least one edge of the top sheet to form a sealed bag, wherein the polymer material is formed from a composition comprising:
A. a copolymer of ethylene and at least one α-olefin; and
B. between 25 and 300 ppm by weight of α-tocopherol relative to the component A.

2. The inflatable bioreactor bag according to claim 1, wherein the composition further comprises:
C. a stearate.

3. The inflatable bioreactor bag according to any one of claims 1-2, wherein the copolymer of ethylene and at least one α-olefin is linear low density polyethylene.

4. The inflatable bioreactor bag according to any one of claims 1-3, wherein the amount of component B ranges between > 50 ppm and <150 ppm by weight relative to the component A.

5. The inflatable bioreactor bag according to any one of claims 2-4, wherein the amount of component C ranges between 100 ppm and 1000 ppm by weight relative to the component A.

6. The inflatable bioreactor bag according to any one of claims 1-5, wherein the top sheet and the bottom sheet are formed from a contiguous sheet folded at least partially upon itself.

7. A bioreactor comprising a plurality of chambers, each of the plurality of chambers comprising a chamber wall and being provided for receiving biomass, each of the plurality of chambers further comprising a chamber inlet for supplying material to be treated to the chamber and a chamber outlet for discharging treated material from the chamber, the plurality of chambers being made of a polymer material that comprises at least one flexible film of a polymer material, wherein the polymer material is formed from a composition comprising:
A. a copolymer of ethylene and at least one α-olefin; and
B. between 25 and 300 ppm by weight of α-tocopherol relative to the component A.

8. The bioreactor according to claim 7, wherein the composition further comprises:
C. a stearate.

9. The bioreactor according to any one of claims 7-8, wherein the copolymer of ethylene and at least one α-olefin is linear low density polyethylene.

10. The bioreactor according to any one of claims 7-9, wherein the amount of component B ranges between > 50 ppm and <150 ppm by weight relative to the component A.

11. The bioreactor according to any one of claims 8-10, wherein the amount of component C ranges between 100 ppm and 1000 ppm by weight relative to the component A.

12. A bioreactor bag comprising:
a top sheet comprising at least a first polymer material layer spaced from a second polymer material layer; and
a bottom sheet comprising at least a third polymer material layer spaced from a fourth polymer material layer, the bottom sheet coupled along at least one edge of the top sheet to form a sealed bag, wherein at least one of the first, second , third, and fourth material layer is formed from a composition comprising:
A. a copolymer of ethylene and at least one α-olefin; and
B. between 25 and 300 ppm by weight of α-tocopherol relative to the component A.

13. The bioreactor bag according to claim 12, wherein the copolymer of ethylene and at least one α-olefin is linear low density polyethylene.

14. The bioreactor bag according to any one of claims 12-13, wherein a gaseous layer is disposed between at least the first polymer material layer and the second polymer material layer.

15. The bioreactor bag according to any one of claims 12-13, wherein a gaseous layer is disposed between at least the third polymer material layer and the fourth polymer material layer.

## Patentansprüche

1. Aufblasbarer Bioreaktorbeutel, umfassend:
eine Oberfolie aus Polymermaterial und
eine Unterfolie aus dem Polymermaterial, die entlang mindestens einer Kante der Oberfolie gekoppelt ist, um einen versiegelten Beutel zu bilden, wobei das Polymermaterial aus einer Zusammensetzung gebildet ist, die Folgendes umfasst:
A. ein Copolymer aus Ethylen und mindestens einem α-Olefin und
B. zwischen 25 und 300 ppm nach Gewicht α-Tocopherol relativ zur Komponente A.

2. Aufblasbarer Bioreaktorbeutel nach Anspruch 1, wobei die Zusammensetzung ferner Folgendes umfasst:
C. ein Stearat.

3. Aufblasbarer Bioreaktorbeutel nach einem der Ansprüche 1 bis 2, wobei das Copolymer aus Ethylen und mindestens einem α-Olefin lineares Polyethylen niedriger Dichte ist.

4. Aufblasbarer Bioreaktorbeutel nach einem der Ansprüche 1 bis 3, wobei die Menge an Komponente B im Bereich zwischen >50 ppm und <150 ppm nach Gewicht relativ zur Komponente A liegt.

5. Aufblasbarer Bioreaktorbeutel nach einem der Ansprüche 2 bis 4, wobei die Menge an Komponente C im Bereich zwischen 100 ppm und 1000 ppm nach Gewicht relativ zur Komponente A liegt.

6. Aufblasbarer Bioreaktorbeutel nach einem der Ansprüche 1 bis 5, wobei die Oberfolie und die Unterfolie aus einer zusammenhängenden Folie, die mindestens teilweise auf sich selbst gefaltet ist, gebildet sind.

7. Bioreaktor, umfassend eine Vielzahl von Kammern, wobei jede der Vielzahl von Kammern eine Kammerwand umfasst und zum Aufnehmen von Biomasse bereitgestellt ist, wobei jede der Vielzahl von Kammern ferner einen Kammereinlass zum Zuführen von zu behandelndem Material in die Kammer und einen Kammerauslass zum Abführen von behandeltem Material aus der Kammer umfasst, wobei die Vielzahl von Kammern aus einem Polymermaterial hergestellt ist, das mindestens einen flexiblen Film aus einem Polymermaterial umfasst, wobei das Polymermaterial aus einer Zusammensetzung gebildet ist, die Folgendes umfasst:
A. ein Copolymer aus Ethylen und mindestens einem α-Olefin und
B. zwischen 25 und 300 ppm nach Gewicht α-Tocopherol relativ zur Komponente A.

8. Bioreaktorbeutel nach Anspruch 7, wobei die Zusammensetzung ferner Folgendes umfasst:
C. ein Stearat.

9. Bioreaktor nach einem der Ansprüche 7 bis 8, wobei das Copolymer aus Ethylen und mindestens einem α-Olefin lineares Polyethylen niedriger Dichte ist.

10. Bioreaktor nach einem der Ansprüche 7 bis 9, wobei die Menge an Komponente B im Bereich zwischen >50 ppm und <150 ppm nach Gewicht relativ zur Komponente A liegt.

11. Bioreaktor nach einem der Ansprüche 8 bis 10, wobei die Menge an Komponente C im Bereich zwischen 100 ppm und 1000 ppm nach Gewicht relativ zur Komponente A liegt.

12. Bioreaktorbeutel, umfassend:
eine Oberfolie, die mindestens eine erste Polymermaterialschicht umfasst, die von einer zweiten Polymermaterialschicht beabstandet ist, und
eine Unterfolie, die mindestens eine dritte Polymermaterialschicht umfasst, die von einer vierten Polymermaterialschicht beabstandet ist, wobei die Unterfolie entlang mindestens einer Kante der Oberfolie gekoppelt ist, um einen versiegelten Beutel zu bilden, wobei mindestens eine der ersten, zweiten, dritten und vierten Materialschicht aus einer Zusammensetzung gebildet ist, die Folgendes umfasst:
A. ein Copolymer aus Ethylen und mindestens einem α-Olefin und
B. zwischen 25 und 300 ppm nach Gewicht α-Tocopherol relativ zur Komponente A.

13. Bioreaktorbeutel nach Anspruch 12, wobei das Copolymer aus Ethylen und mindestens einem α-Olefin lineares Polyethylen niedriger Dichte ist.

14. Bioreaktorbeutel nach einem der Ansprüche 12 bis 13, wobei eine gasförmige Schicht zwischen mindestens der ersten Polymermaterialschicht und der zweiten Polymermaterialschicht angeordnet ist.

15. Bioreaktorbeutel nach einem der Ansprüche 12 bis 13, wobei eine gasförmige Schicht zwischen mindestens der dritten Polymermaterialschicht und der vierten Polymermaterialschicht angeordnet ist.

## Revendications

1. Sac gonflable de bioréacteur comprenant :
une feuille supérieure de matériau polymère ; et
une feuille inférieure du matériau polymère couplée le long d'au moins un bord de la feuille supérieure pour former un sac scellé, dans lequel le matériau polymère est formé à partir d'une composition comprenant :
A. un copolymère d'éthylène et d'au moins une α-oléfine ; et
B. entre 25 et 300 ppm en poids d'a-tocophérol par rapport au composant A.

2. Sac gonflable de bioréacteur selon la revendication 1, dans lequel la composition comprend en outre :
C. un stéarate.

3. Sac gonflable de bioréacteur selon l'une quelconque des revendications 1 à 2, dans lequel le copolymère d'éthylène et d'au moins une α-oléfine est un polyéthylène linéaire basse densité.

4. Sac gonflable de bioréacteur selon l'une quelconque des revendications 1 à 3, dans lequel la quantité de composant B est comprise entre > 50 ppm et < 150 ppm en poids par rapport au composant A.

5. Sac gonflable de bioréacteur selon l'une quelconque des revendications 2 à 4, dans lequel la quantité de composant C est comprise entre 100 ppm et 1000 ppm en poids par rapport au composant A.

6. Sac gonflable de bioréacteur selon l'une quelconque des revendications 1 à 5, dans lequel la feuille supérieure et la feuille inférieure sont formées à partir d'une feuille contiguë pliée au moins partiellement sur elle-même.

7. Bioréacteur comprenant une pluralité de chambres, chacune de la pluralité de chambres comprenant une paroi de chambre et étant prévue pour recevoir de la biomasse, chacune de la pluralité de chambres comprenant en outre une entrée de chambre pour fournir du matériau à traiter à la chambre et une sortie de chambre pour décharger le matériau traité de la chambre, la pluralité de chambres étant constituées d'un matériau polymère qui comprend au moins un film flexible d'un matériau polymère, dans lequel le matériau polymère est formé à partir d'une composition comprenant :
A. un copolymère d'éthylène et d'au moins une α-oléfine ; et
B. entre 25 et 300 ppm en poids d'a-tocophérol par rapport au composant A.

8. Bioréacteur selon la revendication 7, dans lequel la composition comprend en outre :
C. un stéarate.

9. Bioréacteur selon l'une quelconque des revendications 7 à 8, dans lequel le copolymère d'éthylène et d'au moins une α-oléfine est un polyéthylène linéaire basse densité.

10. Bioréacteur selon l'une quelconque des revendications 7 à 9, dans lequel la quantité de composant B est comprise entre > 50 ppm et < 150 ppm en poids par rapport au composant A.

11. Bioréacteur selon l'une quelconque des revendications 8 à 10, dans lequel la quantité de composant C est comprise entre 100 ppm et 1000 ppm en poids par rapport au composant A.

12. Sac de bioréacteur comprenant :
une feuille supérieure comprenant au moins une première couche de matériau polymère espacée d'une seconde couche de matériau polymère ; et
une feuille inférieure comprenant au moins une troisième couche de matériau polymère espacée d'une quatrième couche de matériau polymère, la feuille inférieure étant couplée le long d'au moins un bord de la feuille supérieure pour former un sac scellé, dans lequel au moins l'une des première, seconde, troisième, et quatrième couche de matériau est formée à partir d'une composition comprenant :
A. un copolymère d'éthylène et d'au moins une α-oléfine ; et
B. entre 25 et 300 ppm en poids d'a-tocophérol par rapport au composant A.

13. Sac de bioréacteur selon la revendication 12, dans lequel le copolymère d'éthylène et d'au moins une α-oléfine est un polyéthylène linéaire basse densité.

14. Sac de bioréacteur selon l'une quelconque des revendications 12 à 13, dans lequel une couche gazeuse est disposée entre au moins la première couche de matériau polymère et la seconde couche de matériau polymère.

15. Sac de bioréacteur selon l'une quelconque des revendications 12 à 13, dans lequel une couche gazeuse est disposée entre au moins la troisième couche de matériau polymère et la quatrième couche de matériau polymère.
